# EUROPEAN PATENT APPLICATION

(11) **EP 2 106 198 A1**
(43) Date of publication of application: **30.09.2009**
(21) Application number: 07849919.1
(22) Date of filing: 29.11.2007
(51) Int. Cl.: H05B 37/02, A61M 21/02, A61N 5/06

(54) **LIGHT SOURCE AND LIGHT IRRADIATING DEVICE**

(30) Priority: 08.12.2006 JP 2006331940
(71) Applicant: Sharp Kabushiki Kaisha, Osaka-shi, Osaka 545-8522 (JP)
(72) Inventor: TSUBOI, Masanori, Osaka-shi, Osaka 545-8522 (JP); UCHIUMI, Tadashi, Osaka-shi, Osaka 545-8522 (JP); KANEKO, Takashi, Osaka-shi, Osaka 545-8522 (JP); YAMANAKA, Atsushi, Osaka-shi, Osaka 545-8522 (JP)
(74) Representative: Müller - Hoffmann & Partner
(86) International application number: PCT/JP2007/073088
(87) International publication number: WO 2008/069103

(57) **Abstract**

A light source and a light irradiating device are provided to efficiently regulate a biological rhythm of a user without changing a color temperature of illumination light, an average color-matching index (Ra) or both. The light irradiating device is comprised of a blue emitter (11) that emits light with major energy in a wavelength region adjacent to a wavelength to minimize a value of M(λ)/z(λ), where M(λ) is a relative sensitivity in an antisecretory characteristic of melatonin and z(λ)is a relative sensitivity of a color matching function, a blue emitter (12) that emits light with major energy in a wavelength different from the wavelength of the blue emitter (11), a red emitter (13), a green emitter (14), a diffusing plate (15) that diffuses light from each emitter, a controller (16); that controls the light emission intensity of each emitter, an information input device (17) that inputs information on adjustment of the degree of biological reaction of each emitter, information on an illumination color temperature and the like, a switch (18) that can turn on or off each emitter, and a memory device (19) that stores the light emission intensity of each emitter and its adjusting methods.

## Description

### TECHNICAL FIELD

The present invention relates to a light source and a light irradiating device, and, more particularly, to a light source and a light irradiating device that are capable of adjusting the biological rhythm of a user.

### BACKGROUND OF THE INVENTION

It is known that a person has day-cycle biological rhythms (circadian rhythm) that control cyclic phenomena related to biofunctions. Although the cycle of a biological rhythm is actually slightly longer than 24 hours, being exposed to light in the morning advances the phase of the biological rhythm to adjust the biological rhythm to the 24-hour cycle living environment.

For example, the rhythm of sleep and awakening is one of typical biological rhythms, and melatonin secretion is deeply related to this rhythm. Melatonin is a hormone secreted from the pineal body of the brain. This hormone is usually secreted at night in a time zone between pre-hypnagogic time and the first half of sleep, being considered to be a hormone that contributes to acceleration of a body temperature decrease and falling asleep. It is known that being exposed to relatively intensive light in the nighttime, especially in a time zone before going to bed, inhibits the secretion of melatonin. It is also known, conversely, that being exposed to relatively intensive light in the daytime increases an amount of secretion of melatonin in the nighttime. Light is, therefore, greatly concerned with adjustment of the biological rhythm, and light irradiating time zone and light intensity are particularly important factors in biological rhythm adjustment.

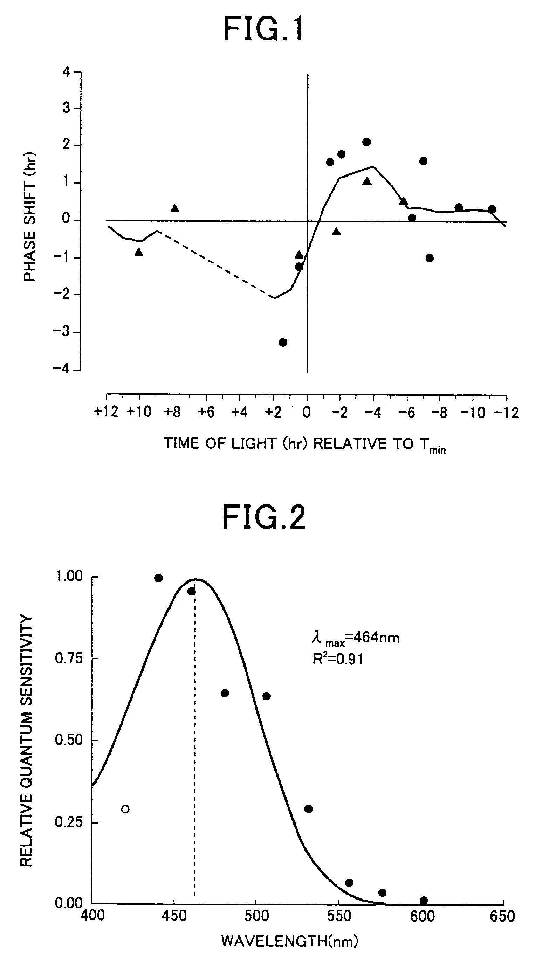

Fig. 1 depicts a phase response curve of the biological rhythm of a person (Nonpatent Literature 1). In Fig. 1, the horizontal axis represents the time of appearance of the person's lowest body temperature as zero, the time before the lowest body temperature appearance time as positive values, and the time after the lowest body temperature appearance time as negative values, while the vertical axis represents a time span during which the phase of the biological rhythm advances and a time span during which the phase of the biological rhythm retreats as positive values and negative values, respectively. For example, Fig. 1 demonstrates that light irradiating approximately four hours after the lowest body temperature appearance time advances the phase of the biological rhythm by about one and half hours, while light irradiating approximately two hours before the lowest body temperature appearance time retreats the phase of the biological rhythm by about two hours.

It is clearly known that the characteristics of melatonin secretion inhibition vary depending on light wavelengths. Fig. 2 depicts the wavelength characteristics of melatonin secretion inhibition caused by exposure to light in the nighttime (Nonpatent Literature 2). In Fig. 2, the horizontal axis represents light wavelength [nm], while the vertical axis represents relative sensitivity. In Fig. 2, the relative sensitivity for melatonin secretion inhibition becomes the highest at a wavelength of 464 [nm]. Light having much energy in this wavelength range, therefore, shows a larger melatonin secretion inhibiting effect.

An irradiating method and an irradiating device described in Patent Document 1 have been devised as an apparatus that adjusts the biological rhythm in use of the above knowledge. The method and apparatus relate to a light irradiating method and a light irradiating device that take into consideration light irradiating time zone and light spectrum based on the biological rhythm phase response characteristics of Fig. 1 and the melatonin secretion inhibition characteristics of Fig. 2.

With respect to light irradiating time zone, according to the irradiating method and irradiating device, a time zone ranging from a lowest body temperature appearance time to a time at least 11 hours after the lowest body temperature appearance time is determined to be an irradiating recommendation time zone. This is because that light irradiating in the period from the lowest body temperature appearance time to a time about 11 hours after the lowest body temperature appearance time advances the phase of the biological rhythm, thus facilitating adjustment of the biological rhythm to the 24-hour cycle while keeping a daytime alertness high to improve the quality of nighttime sleep. Light irradiating in the irradiating recommendation time zone is, therefore, effective in adjusting the biological rhythm.

In contrast, a time zone ranging from a time about five hours after the end time of an irradiating recommendation time zone to the start time of the next irradiating recommendation time zone is determined to be an irradiating suspension time zone. This is because that light irradiating in the period from a time about eight hours before the lowest body temperature appearance time to the lowest body temperature appearance time retreats the phase of the biological rhythm to invite a deterioration in the quality of nighttime sleep. The above time zone, therefore, is determined to be the irradiating suspension time zone.

With respect to the spectrum of a light source to be used, a light source having the maximum peak in a wavelength range of 410[nm] to 505 [nm] covering wavelengths effective for melatonin secretion inhibition and an ordinary white light source are used. The former light source is used in the irradiating recommendation time zone for irradiating at a given illumination level and in a given illumination time that are necessary for adjustment of the biological rhythm. The latter light source is used in the irradiating suspension time zone.

As described above, by the light irradiating method of using time zones for light irradiating and light sources emitting light having different spectra for separate purposes, the advance of phase of the biological rhythm is facilitated efficiently to improve an alertness in the daytime, while inhibition of melatonin secretion is avoided to prevent the retreat of phase of the biological rhythm in the nighttime. The biological rhythm is thus adjusted in this manner.
Patent Document 1: Japanese Laid-Open Patent Publication No. 2005-310654
Nonpatent Literature 1: "A human phase shift curve to light", Minors et al, 1991
Nonpatent Literature 2: "Action Spectrum for Melatonin Regulation in Humans: Evidence for a Novel Circadian Photoreceptor", Brainerd et al, 2001

### DISCLOSURE OF THE INVENTION

### PROBLEMS TO BE SOLVED BY THE INVENTION

As described above, adjustment of the biological rhythm can be carried out efficiently by using light sources having different spectra for irradiating and changing a time of use of each of the light sources depending on a time zone. Because the light sources have spectra different from each other, however, irradiating light comes to have color temperatures different from each other.

For example, when irradiating is carried out consecutively from an irradiating suspension time zone to an irradiating recommendation time zone, a light source on use is changed as the time zone is changed. As a result, the color temperature of irradiating light is also changed, which gives a user a feeling of oddness.

A change in a color temperature leads to a change in the value of a general color rendering index, which is a typical numerical value that represents how faithfully an object color reproduces the original color as a result of illumination. The higher the general color rendering index is, the better color rendering properties are. The general color rendering index is given by averaging differences between the colors of eight reference samples that result when the samples are irradiated with irradiating light to be assessed and the colors of the reference samples that result when the samples are irradiated with reference light, and is affected by the spectrum of irradiating light. If a spectrum is different, therefore, how an object color looks is different, which gives the user a feeling of oddness.

The present invention was conceived in view of the above problems, and it is therefore the object of the present invention to provide a light source and a light irradiating device that are capable of efficiently adjusting the biological rhythm of a user without changing either or both of the color temperature and the general color rendering index of irradiating light.

### MEANS FOR SOLVING THE PROBLEMS

A light source of the present invention is provided for solving the above problem. When a relative sensitivity in the wavelength characteristics of melatonin secretion inhibition is M(λ) and a relative sensitivity on a color matching function for human eyesight is z(λ), the light source of the present invention includes a blue emitter that has a large quantity of energy or a peak in a wavelength range near a wavelength that makes M(λ)/z(λ) the minimum, and a blue emitter that has a large quantity of energy or a peak in a wavelength range other than the above wavelength range. The light source of the present invention is capable of adjusting the light emission intensity of each of the two types of blue emitters.
An emitter that emits light of a wavelength range of 430 [nm] to 470 [nm] can be used as the blue emitter that emits light of a wavelength range near the wavelength that makes M(λ)/z(λ) the minimum. An emitter that emits light of a wavelength range of 380 [nm] to 440 [nm] and of 460 [nm] to 510 [nm] can be used as the blue emitter that emits light of a wavelength range other than the above wavelength range.

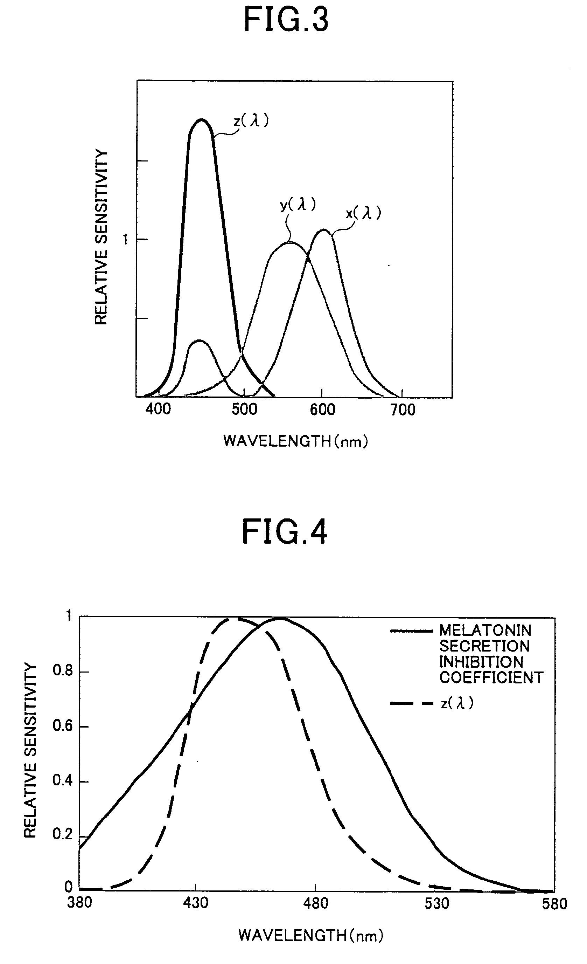

Fig. 3 depicts color matching functions for equal energy in the XYZ color specification system. These color matching functions are given by converting color matching functions in the RGB color specification system representing the ratio of mixing of the original stimuli (monochromatic light) [R], [G] and [B] necessary for color matching to certain monochromatic light, into color matching functions in the XYZ color specification system. In other words, Fig. 3 depicts the mixing ratio of the original stimuli [X], [Y] and [Z] necessary for reproducing specific monochromatic light. A light wavelength range effective for adjustment of the biological rhythm is a short wavelength range, as described above, and the original stimulus corresponding to the short wavelength range is the original stimulus [Z]. Attention is now directed, therefore, to a color matching function z(λ).

Fig. 4 is a diagram of the melatonin secretion inhibition characteristics M(λ) of Fig. 2 and the color matching function z(λ) of Fig. 3 that are plotted on the same scale with the maximum relative sensitivity on the characteristics M(λ) and on the function z(λ) set to 1. Fig. 4 shows that the relative sensitivity on the color matching function z(λ) is high near a wavelength range of 430 to 470 [nm].

For example, blue light having a peak at a wavelength of 450 [nm] that makes the value of z(λ) the maximum and carrying relative energy of 1 is reproduced. The value of M(λ) when the value of z(λ) is at its maximum is about 0.9. Light identical with this blue light is then reproduced using light of a wavelength of 480 [nm]. In this case, the value of z(λ) is about 0.5 at the wavelength of 480 [nm], which, in simple calculation, makes necessary relative energy two times the relative energy of light of the wavelength of 450 [nm]. As a result, the value of M(λ) is also doubled to become 1.8 that is two times 0.9 as a numerical value. This means that light of the wavelength of 480 [nm] has a greater effect on melatonin secretion inhibition.

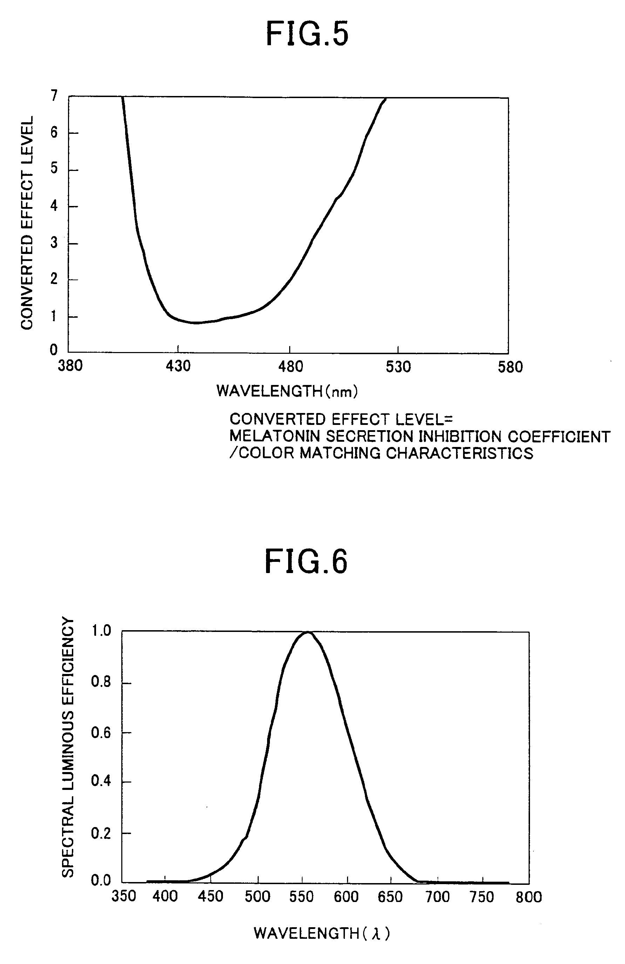

Fig. 5 is a diagram of M(λ) /z(λ) depicted as a converted effect level, the M(λ)/z(λ) representing the ratio of the melatonin secretion inhibition characteristics M(λ) to the color matching function z(λ). Based on this converted effect level, when blue light having a specific chromaticity is reproduced using light having much energy near a wavelength that makes the converted effect level the minimum and light having much energy in a wavelength range not near the wavelength that makes the converted effect level the minimum, the intensity of each light can be determined. In other words, an effect on melatonin secretion inhibition can be changed as the chromaticity is kept constant, which enables adjustment of the biological rhythm of the user.

The light source of the present invention includes the blue emitter, a red emitter, and a green emitter. The light source of the present invention is capable of adjusting the light emission intensity of each of the blue emitter, the red emitter, and the green emitter, thus capable of forming white light through light emission intensity adjustment to keep the color temperature of the white light constant. The light source of the present invention is also capable of adjusting an effect on melatonin secretion inhibition. This enables effective adjustment of the biological rhythm of the user in use of light with a constant color temperature.

Adjustment of the color temperature and the level of effect on melatonin secretion inhibition (this influence is also called "bioeffect level" or "biological effect") of white light is carried out by a method of optimization analysis through numerical value calculation. For example, a case of determining the light emission intensity of each of the emitters that maximizes a bioeffect level at a specific color temperature is assumed. In this case, the light emission intensity of each of the emitters is treated as a variable and the color temperature of white light to be formed is set constant under a specific condition. In addition, a precondition that a light emission intensity variable is equal to or more than zero is set as an additional condition, and numerical value calculation is carried out to maximize the bioeffect level in execution of the optimization analysis. Hence the light emission intensity of each of the emitters as a variable can be determined.

A method for determining a bioeffect level will be described. A bioeffect level is determined by using a concept of expressing light in terms of energy quantity.

When light is expressed in terms of energy, light can be expressed as radiant flux, which indicates the radiant energy that passes through a certain plane in each unit time. The energy of light emitted on each unit area is referred to as irradiance. These concepts represent physical quantity that takes into consideration space, time, and wavelengths in expressing energy quantity carried by light radiating into a space, thus referred to as radiometric quantity. Radiometric quantity, however, is defined without considering how light looks to human eyes. For example, such light as infrared carries much energy but is not visible light, thus invisible to people.

Conceptional application of the human eyes' sensitivity to light to radiometric quantity gives a concept of photometric quantity. Photometric quantity corresponds in unit to radiometric quantity. For example, luminous flux is defined by considering the human eyes' sensitivity in expressing radiant flux, and illuminance is defined by considering the human eyes' sensitivity in expressing irradiance. A concept called luminous efficacy is defined with regard to the human eyes' sensitivity, and luminous efficacy varies depending on wavelengths. When seeing an object in a place with sufficient light quantity, a person feels light of a wavelength of 555[nm] to be the brightest. The luminous efficacy at this wavelength is referred to as maximum luminous efficacy. The maximum luminous efficacy is defined as 1 while luminous efficacies at other wavelengths are expressed relative to the maximum luminous efficacy to provide a concept referred to as spectral luminous efficacy. The spectral luminous efficacy standardized by Commission Internationale de l'Eclairage is referred to as standard spectral luminous efficacy.

Fig. 6 is a graph of the standard spectral luminous efficacy plotted against wavelength that is referred to as a standard spectral luminous efficacy curve. Fig. 6 demonstrates that the human eyes' sensitivity to light wavelength becomes the highest at a wavelength of 555 [nm], from which peak the sensitivity declines. A luminous flux is given by multiplying together a spectral luminous efficacy, a radiant flux, and the maximum luminous efficacy (value that correlates photometric quantity with radiometric quantity) at each wavelength and summing up, i.e., integrating multiplication results in a visible light range.

Photometric quantity, however, represents the energy that is defined by considering how light looks to the human eyes, and is, therefore, not defined by considering an effect on melatonin secretion inhibition. The effect on melatonin secretion inhibition is indicated by the melatonin secretion inhibition characteristics of Fig. 2, as described above. The melatonin secretion inhibition characteristics are thus utilized in replacement of the standard spectral luminous efficacy. Specifically, the value of relative sensitivity in the melatonin secretion inhibition characteristics and a radiant flux at each wavelength are multiplied together and multiplication results are summed up, i.e., integrated in the visible light range to produce a value. This value is equivalent to a numerical value that represents an effect on melatonin secretion inhibition, and is used as a bioeffect level. A bioeffect level is thus determined by the above method.

A light irradiating device of the present invention is capable of adjusting the light emission intensity of each of emitters so that the general color rendering index of white light formed by light emission intensity adjustment does not become lower than a given value. Even when the general color rendering index is taken into consideration, light emission intensity adjustment is carried out using the above method of optimization analysis. Specifically, for example, a target general color rendering index is set as an additional condition, and a bioeffect level is determined by numerical calculation under the condition. Through this method, the general color rendering index is kept from becoming lower than the given value at the same color temperature, and luminance is also kept at a given value through the same method. Hence the above method enables adjusting the biological rhythm of the user without giving the user a feeling of oddness.

By the above means, according to the present invention, the biological rhythm of the user can be adjusted efficiently as white light is formed by adjusting the light emission intensity of each of the blue emitter, red emitter, and the green emitter, the color temperature of the white light is kept constant, and the general color rendering index is kept from becoming lower than the given value. Hence irradiating light not giving the user a feeling of oddness can be provided.

### Effect of the Invention

The present invention enables keeping a color temperature constant and keeping a general color rendering index from becoming lower than a given value even when a biological rhythm adjustment effect is changed, thus enables effectively adjusting the biological rhythm without giving a user a feeling of oddness.

### BRIEF DESCRIPTION OF DRAWINGS

[Fig. 1] Fig. 1 is a graph showing that at what degree the phase of the human biological rhythm shifts depending on light irradiating time zones;
[Fig. 2] Fig. 2 is a graph of the relation between a light wavelength and the magnitude of an effect on melatonin secretion;
[Fig. 3] Fig. 3 is adiagramof colormatching functions in the XYZ color specification system;
[Fig. 4] Fig. 4 is a diagram of the graph of the relation between a light wavelength and the magnitude of an effect on melatonin secretion and the color matching function z(λ) in the XYZ color specification system that are plotted on the same scale with the maximum relative sensitivity on the graph and on the function z(λ) set to 1;
[Fig. 5] Fig. 5 is a diagram of M(λ)/z(λ) depicted as a converted effect level, M(λ)/z(λ) representing the ratio of the graph M(λ) of the relation between a light wavelength and the magnitude of an effect on melatonin secretion to the color matching function z(λ);
[Fig. 6] Fig. 6 is a graph of a standard spectral luminous efficacy curve;
[Fig. 7] Fig. 7 is a diagram of a light irradiating device according to a first embodiment of the present invention;
[Fig. 8] Fig. 8 is a diagram of an example of the spectrum of white light of which the bioeffect level is reduced to an extremely small one under a condition that the color temperature, the general color rendering index, and the luminance are constant;
[Fig. 9] Fig. 9 is a diagram of an example of the spectrum of white light of which the bioeffect level is enlarged to an extremely large one under a condition that the color temperature, the general color rendering index, and the luminance are constant;
[Fig. 10] Fig. 10 is a diagram of a light irradiating device according to a second embodiment of the present invention;
[Fig. 11] Fig. 11 is a diagram of a light irradiating device according to a third embodiment of the present invention; and
[Fig. 12] Fig. 12 is a diagram of a light irradiating device according to a fourth embodiment of the present invention.

### EXPLANATION OF REFERENCE NUMERALS

- 11: blue emitter
- 12: blue emitter
- 13: red emitter
- 14: green emitter
- 15: diffuser plate
- 16: controller
- 17: information input device
- 18: switch
- 19: memory device
- 21: arm
- 31: enclosure
- 32: liquid crystal panel

### PREFERRED EMBODIMENTS OF THE INVENTION

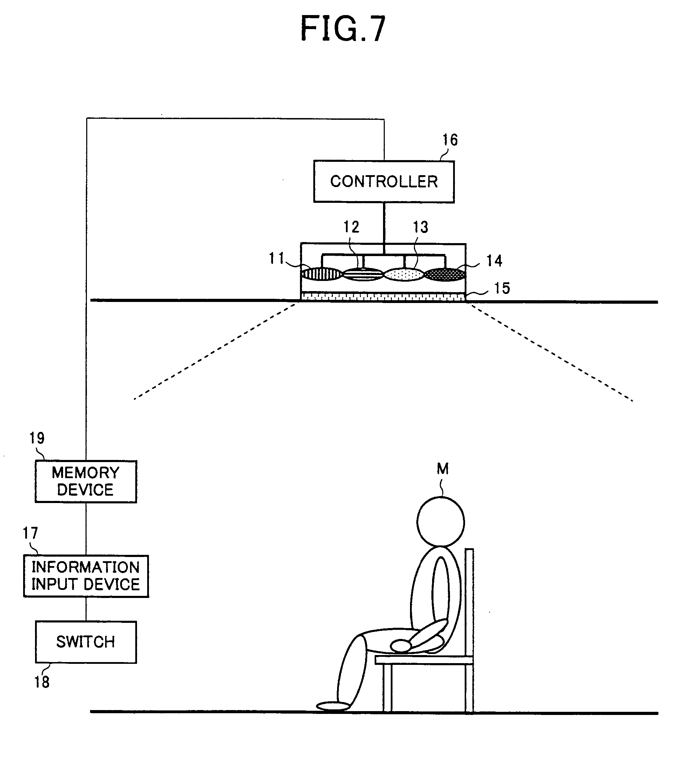

Fig. 7 is a diagram of a light irradiating device according to a first embodiment of the present invention. In this embodiment, a relative sensitivity in melatonin secretion inhibition characteristics is denoted by M(λ) and a relative sensitivity on a color matching function for human eyesight is denoted by z(λ). The light irradiating device of this embodiment includes a blue emitter 11 that emits light having much energy in a wavelength range near a wavelength that makes M(λ)/z(λ) the minimum, a blue emitter 12 that has much energy in a wavelength range different from the wavelength range of the blue emitter 11, a red emitter 13 that emits red light, a green emitter 14 that emits green light, a diffuser plate 15 that diffuses light emitted from the emitters 11 to 14, a controller 16 that controls the light emission intensities of the emitters 11 to 14, an information input device 17 that inputs information on adjustment of the bioeffect levels of the emitters 11 to 14, information of irradiating light color temperatures, etc., a switch 18 capable of turning on and off the emitters in switching operation, and a memory device 19 having stored thereon the light emission intensity of each of the emitters and a method of adjusting the light emission intensity.

In this embodiment, the light irradiating device is applied to an indoor illumination. A user M inputs information for bioeffect level adjustment using the information input device 17 to be able to be exposed to light effective for adjustment of the biological rhythm. The user is able to turn on and off irradiating light in switching operation using the switch 18.

Information input to the information input device 17 is assumed to be information on the numerical value of a color temperature and the strongness/weakness of a bioeffect level, information of luminance, etc., but is not limited to these types of information. Information of the color temperature may be input as the name of a light source color that is attached to an illumination currently in popular use, such as daytime white and daylight color, and such pieces of name information may be selectable, which means that any information specifying the color temperature is applicable. Information on the strongness/weakness of the bioeffect level, for example, may be input directly as a numerical value representing the bioeffect level, or may be input as a value that is selected from preset numerical values. Information of the bioeffect level may not be given as a numerical value, but may be given as a linguistic expression using such words as strong and weak that indicates bioeffect levels step by step, from which a bioeffect level to input can be selected. Any information specifying the bioeffect level is thus applicable. Likewise, any information specifying the luminance is applicable in inputting information of the luminance.

Because keeping an alertness high in the daytime while not keeping an alertness higher than a necessary level in the nighttime is effective for adjustment of the biological rhythm, timing of emitting light having a high bioeffect level in the daytime and emitting light having low bioeffect level in the nighttime may be stored in advance in the memory device 19 to automatically adjust the light emission intensity of each of the emitters without input through the information input device 17. Time information may be stored in advance in the memory device 19 to adjust the light emission intensity of each of the emitters using the stored information. A light emission intensity pattern of each of the emitters may be stored in advance in the memory device 19 to automatically adjust the light emission intensity using the stored light emission intensity pattern. The emitters 11 to 14 may not be used one by one as shown in Fig. 7, but a plurality of the emitters may be used regardless of the types of the emitters. While light is diffused by the diffuser plate 15, a method of light diffusion is not limited to this method. Any light diffusion method is applicable if the method provides the user M with light available as illumination.

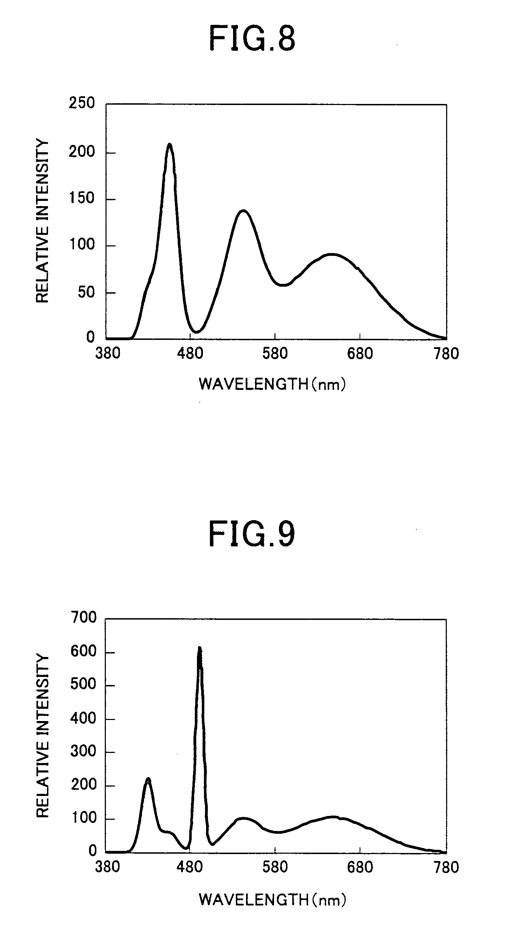

Fig. 8 depicts an example of the spectrum of white light formed by the emitter having a light emission intensity that is determined to keep the color temperature and luminance constant, to make the general color rendering index equal to or more than a given value, and to reduce the bioeffect level to extremely small one. Fig. 9 depicts an example of the spectrum of white light of which the bioeffect level is extremely large. The spectra of both types of white light are different, which is clearly shown in Figs. 8 and 9, but the color temperatures and the general color rendering indexes of both light are substantially the same as the value of the general color rendering index is kept equal to or more than a given value.

While the spectrum of white light formed in this embodiment is adjusted to the spectra shown in Fig. 8 and in Fig. 9 by controlling the light emission intensity of each of the emitters 11 to 14 with the controller 16, spectrum adjustment is not limited to this case. For example, the spectrum may be adjusted so that the bioeffect level becomes different from the bioeffect levels of Figs. 8 and 9 as the color temperature and the general color rendering index are substantially the same as those of Figs. 8 and 9. In another case, the bioeffect level may be adjusted at a color temperature different from the color temperature of the spectra of Figs. 8 and 9, or the value of the general color rendering index may be increased.

While the above light irradiating device is described as an indoor illumination, the application form of the light irradiating device is not limited to this. For example, the light irradiating device may apply to an illumination in a traveling body, such as an interior light in a car or a train.

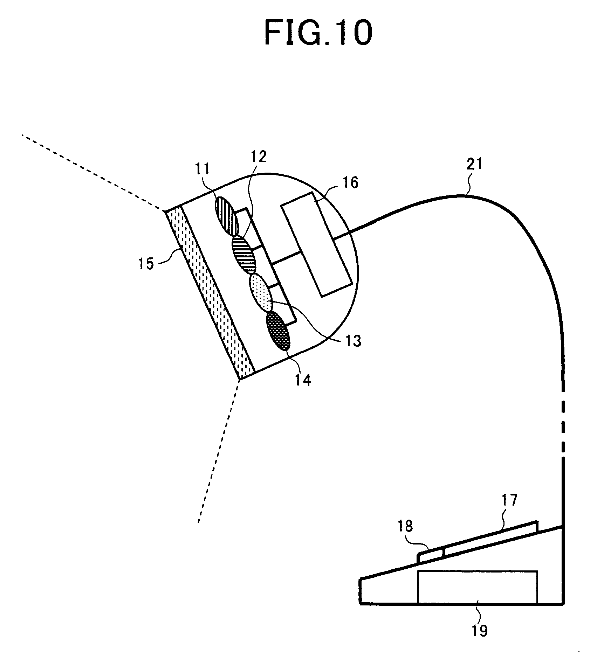

Fig. 10 is a diagram of a light irradiating device according to a second embodiment of the present invention. The light irradiating device of this embodiment includes the blue emitters 11 and 12 that emit blue light, the red emitter 13, the green emitter 14, the diffuser plate 15, the controller 16, the information input device 17, the switch 18, the memory device 19, and an arm 21 that supports the emitters 11 to 14, the diffuser plate 15, and the controller. Each of the blue emitters 11 and 12, the red emitter 13, the green emitter 14, the diffuser plate 15, the controller 16, the information input device 17, the switch 18, and the memory device 19 has a function identical with the function of each of the same components of Fig. 7, so that the description of the function will be omitted.

In this embodiment, the light irradiating device is applied to a desk lamp that irradiates the surroundings of the hands of a user. The user inputs information for bioeffect level adjustment using the information input device 17 to be able to irradiate a specific spot with light effective for adjustment of the biological rhythm. The user is able to turn on and off irradiating light in switching operation using the switch 18.

Information input to the information input device 17 is assumed to be information on the numerical value of a color temperature and the strongness/weakness of a bioeffect level, information of luminance, etc., but is not limited to these types of information. Information of the color temperature may be input as the name of a light source color that is attached to an illumination currently in popular use, such as daytime white and daylight color, and such pieces of name information may be set selectable, which means that any information specifying the color temperature is applicable. Information on the strongness/weakness of the bioeffect level, for example, may be input directly as a numerical value representing the bioeffect level, or may be input as a value that is selected from preset numerical values. Information of the bioeffect level may not be given as a numerical value, but may be given as a linguistic expression using such words as strong and weak that indicates bioeffect levels step by step, from which a bioeffect level to input can be selected. Any information specifying the bioeffect level is thus applicable. Likewise, any information specifying the luminance is applicable in inputting information of the luminance.

Because keeping an alertness high in the daytime while not keeping an alertness higher than a necessary level in the nighttime is effective for adjustment of the biological rhythm, timing of emitting light having a high bioeffect level in the daytime and emitting light having low bioeffect level in the nighttime may be stored in advance in the memory device 19 to automatically adjust the light emission intensity of each of the emitters without input through the information input device 17. A light emission intensity pattern of each of the emitters may be stored in advance in the memory device 19 to automatically adjust the light emission intensity using the stored light emission intensity pattern. Time information may be stored in advance in the memory device 19 to adjust the light emission intensity of each of the emitters using the stored information. The emitters 11 to 14 may not be used one by one as shown in Fig. 10, but a plurality of the emitters may be used regardless of the types of the emitters. While light is diffused by the diffuser plate 15, a method of light diffusion is not limited to this method. Any light diffusion method is applicable if the method provides the user with light available as illumination.

While the above light irradiating device is described as a desk lamp, the application form of the light irradiating device is not limited to this. For example, the light irradiating device may be used as any form of an illumination that irradiates a personal space, such as a reading lamp.

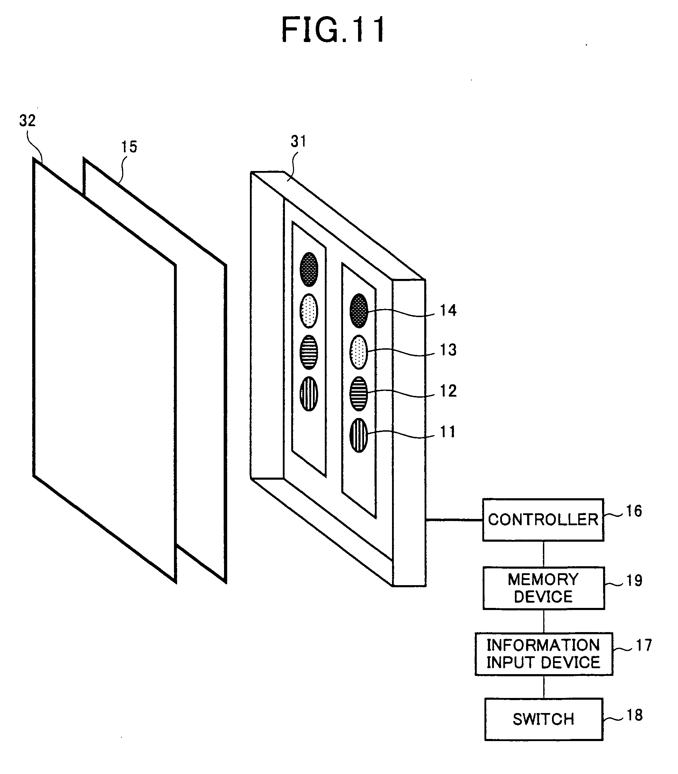

Fig. 11 is a diagram of a light irradiating device according to a third embodiment of the present invention. The light irradiating device of this embodiment includes the blue emitters 11 and 12 that emit blue light, the red emitter 13, the green emitter 14, the diffuser plate 15, the controller 16, the information input device 17, the switch 18, the memory device 19, an enclosure 31 that holds the emitters 11 to 14, and a liquid crystal panel 32 (display means) that displays information of an image, etc. Each of the blue emitters 11 and 12, the red emitter 13, the green emitter 14, the diffuser plate 15, the controller 16, the information input device 17, the switch 18, and the memory device 19 has a function identical with the function of each of the same components of Fig. 7, so that the description of the function will be omitted.

In this embodiment, the light irradiating device is applied to a display apparatus. A user inputs information for bioeffect level adjustment using the information input device 17 to be able to be exposed to light effective for adjustment of the biological rhythm via the display apparatus. The user is able to turn on and off irradiating light in switching operation using the switch 18.

Information input to the information input device 17 is assumed to be information on the numerical value of a color temperature and the strongness/weakness of a bioeffect level, information of luminance, etc., but is not limited to these types of information. Information of the color temperature may be input as the name of a light source color that is attached to an illumination currently in popular use, such as daytime white and daylight color, and such pieces of name information may be set selectable, which means that any information specifying the color temperature is applicable. Information on the strongness/weakness of the bioeffect level, for example, may be input directly as a numerical value representing the bioeffect level, or may be input as a value that is selected from preset numerical values. Information of the bioeffect level may not be given as a numerical value, but may be given as a linguistic expression using such words as strong and weak that indicates bioeffect levels step by step, from which a bioeffect level to input can be selected. Any information specifying the bioeffect level is thus applicable. Likewise, any information specifying the luminance is applicable in inputting information of the luminance.

Because keeping an alertness high in the daytime while not keeping an alertness higher than a necessary level in the nighttime is effective for adjustment of the biological rhythm, timing of emitting light having a high bioeffect level in the daytime and emitting light having low bioeffect level in the nighttime may be stored in advance in the memory device 19 to automatically adjust the light emission intensity of each of the emitters without input through the information input device 17. A light emission intensity pattern of each of the emitters may be stored in advance in the memory device 19 to automatically adjust the light emission intensity using the stored light emission intensity pattern. Time information may be stored in advance in the memory device 19 to adjust the light emission intensity of each of the emitters using the stored information. The emitters 11 to 14 may not be used one by one as shown in Fig. 11, but a plurality of the emitters may be used regardless of the types of the emitters. While light is diffused by the diffuser plate 15, a method of light diffusion is not limited to this method. Any light diffusion method is applicable if the method provides light usable in an irradiating device. The liquid crystal panel 32 (display means) is not limited to the form of a liquid crystal panel, but may be provided as a means capable of displaying information. The controller 16, the switch 18, and the memory device 19 may be housed in the enclosure 31 in any form of arrangement.

While the above light irradiating device is described as a display apparatus, the application form of the light irradiating device is not limited to this. For example, the light irradiating device may apply to a TV set, PC monitor, cellular phone, etc. For example, when the light irradiating device is used in a TV set, a remote controller enables various operations. The information input device 17, the switch 18, etc. , therefore, may be incorporated in the remote controller. When the light irradiating device is used as a PC monitor, the controller 16, the memory device 19, etc., may be incorporated in a PC, or a control function and a memory function of the PC may be used. When the light irradiating device is used in a cellular phone, the controller 16 may be incorporated in the cellular phone, or the components of the cellular phone equivalent to the information input device 17, the switch 18, and the memory device 19 may be used.

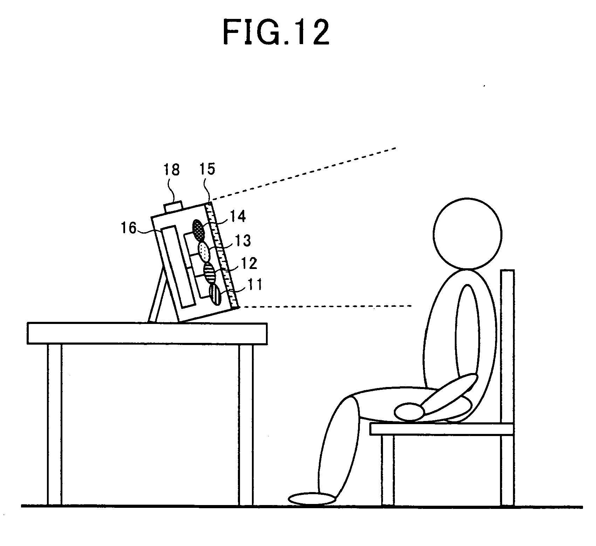

Fig. 12 is a diagram of a light irradiating device according to a fourth embodiment of the present invention. The light irradiating device of this embodiment includes the blue emitters 11 and 12 that emit blue light, the red emitter 13, the green emitter 14, the diffuser plate 15, the controller 16, and the switch 18. Each of the blue emitters 11 and 12, the red emitter 13, the green emitter 14, the diffuser plate 15, the controller 16, and the switch 18 has a function identical with the function of each of the same components of Fig. 7, so that the description of the function will be omitted.

In this embodiment, the light irradiating device is applied to an irradiating device that emits light directly onto a user to adjust the user's biological rhythm. The user is exposed to light effective for adjustment of the biological rhythm. The user is able to turn on and off irradiating light in switching operation using the switch 18.

Since light effective for adjustment of the biological rhythm is light having a high bioeffect level, the irradiating device is basically provided as an apparatus that emits such light. When the bioeffect level of irradiating light is changed in strongness/weakness or the color temperature or luminance of irradiating light is changed, however, the irradiating device may be equipped with the information input device and memory device as separate components. In such a case, information input to the information input device is assumed to be information on the numerical value of a color temperature and the strongness/weakness of a bioeffect level, etc., but is not limited to these types of information. Information of the color temperature may be input as the name of a light source color that is attached to an illumination currently in popular use, such as daytime white and daylight color, and such pieces of name information may be set selectable, which means that any information specifying the color temperature is applicable. Information on the strongness/weakness of the bioeffect level, for example, may be input directly as a numerical value representing the bioeffect level, or may be input as a value that is selected from preset numerical values. Information of the bioeffect level may not be given as a numerical value, but may be given as a linguistic expression using such words as strong and weak that indicates bioeffect levels step by step, from which a bioeffect level to input can be selected. Any information specifying the bioeffect level is thus applicable. Likewise, any information specifying the luminance is applicable in inputting information of the luminance.

The emitters 11 to 14 may not be used one by one as shown in the light irradiating device of Fig. 12, but a plurality of the emitters may be used regardless of the types of the emitters. Four or more types of emitters may be used. While light is diffused by the diffuser plate 15, a method of light diffusion is not limited to this method. Any light diffusion method is applicable if the method allows the biological rhythm adjusting light irradiating device to provide the user with effective light.

While the above light irradiating device is described as an irradiating device that emits light directly onto a user to adjust the user's biological rhythm, the application form of the light irradiating device is not limited to this. For example, the light irradiating device may be provided as an embedded type irradiating device that is constructed by embedding an irradiating device in a wall, ceiling, floor, etc.

As described above, using the light irradiating device of the first to fourth embodiments of the present invention enables keeping a color temperature constant even when a biological rhythm adjustment effect is changed. This enables effectively adjusting the biological rhythm without giving a user a feeling of oddness. While the embodiments of the present invention have been described in detail referring to the drawings, a specific configuration of the present invention is not limited to the configuration described in the embodiments, but includes a design, etc., that does not deviate from the substance of the present invention.

## Claims

1. A light source comprising:
an emitter that emits light having a large quantity of energy in a range A; and
an emitter that emits light having a large quantity of energy in a range B, wherein
the range A represents a wavelength range of 430 [nm] to 470 [nm] and the range B represents a wavelength range of 380 [nm] to 440 [nm] and that of 460 [nm] to 510 [nm].

2. A light source comprising:
an emitter that emits light having a peak at least in a range A; and
an emitter that emits light having a peak at least in a range B, wherein
the range A represents a wavelength range of 430 [nm] to 470 [nm] and the range B represents a wavelength range of 380 [nm] to 440 [nm] and that of 460 [nm] to 510 [nm].

3. A light source comprising the light source as defined in claim 1 or 2, wherein
the light source is capable of separately controlling a light emission intensity of each of emitters making up the light source of claim 1 or 2.

4. A light source at least comprising:
a blue emitter that emits light having a large quantity of energy in a wavelength range near a wavelength that makes M(λ)/z(λ) a minimum; and
a blue emitter that emits light having a large quantity of energy in a wavelength range other than the wavelength range, wherein
M(λ) represents a relative sensitivity in melatonin secretion inhibition characteristics, and z(λ) represents a relative sensitivity on a color matching function for human eyesight.

5. The light source as defined in claim 4, wherein
the light source adjusts a light emission intensity of the blue emitter to keep a chromaticity substantially constant, and adjusts a biological rhythm of a user based on melatonin secretion inhibition characteristics of light.

6. The light source as defined in claim 5, further comprising:
a red emitter that emits red light; and
a green emitter that emits green light.

7. The light source as defined in claim 6, wherein
the light source adjusts a light emission intensity of each of the blue emitter, the red emitter, and the green emitter to form white light and keep a color temperature substantially constant, and adjusts a biological rhythm of a user based on melatonin secretion inhibition characteristics of light.

8. The light source as defined in claim 7, wherein
the light source adjusts a light emission intensity of each of the blue emitter, the red emitter, and the green emitter to form white light, and keeps a general color rendering index of the white color from becoming lower than a given value.

9. The light source as defined in claim 7 or 8, wherein
the light source adjusts a light emission intensity of each of the blue emitter, the red emitter, and the green emitter to form white light, and keeps luminance substantially constant.

10. A light irradiating device at least comprising:
the light source of any one of claims 1 to 9;
a controller that controls a light emission intensity of an emitter making up the light source; and
an information input device that inputs information on a biological effect level, a color temperature, luminance, etc., of irradiating light emitted from the light source.
